# EUROPEAN PATENT SPECIFICATION

(11) **EP 3 697 385 B1**
(45) Date of publication and mention of the grant of the patent: **10.11.2021**
(21) Application number: 18822125.3
(22) Date of filing: 15.10.2018
(51) Int. Cl.: A61K 9/127, A61K 9/00, A61K 31/409, A61K 31/555, A61K 31/695, A61K 41/00, A61K 49/00, A61P 35/00, B82Y 5/00, B82Y 30/00, B82Y 40/00

(54) **LIPOSOME DRUG FORM WITH LIGHT-CONVERTING NANOPARTICLES, A METHOD OF ITS PREPARATION AND USE**
LIPOSOMWIRKSTOFF MIT LICHTKONVERTIERENDEN NANOPARTIKELN, VERFAHREN ZU DESSEN HERSTELLUNG UND VERWENDUNG
FORME MÉDICAMENTEUSE LIPOSOMALE AVEC DES NANOPARTICULES DE CONVERSION DE LUMIÈRE, SA MÉTHODE DE PRÉPARATION ET D'UTILISATION

(30) Priority: 16.10.2017 CZ 20170657
(43) Date of publication of application: 26.08.2020
(73) Proprietor: Institute of Physiology Cas, 142020 Prague 4 (CZ)
(72) Inventor: ENGSTOVÁ, Hana, 16000 Prague 6 (CZ); NEKVASIL, Milos, Zizkov 13000 Prague 3 (CZ); JEZEK, Petr, Cakovice 19600 Prague 9 (CZ); POUCKOVÁ, Pavla, 25229 Dobrichovice (CZ)
(74) Representative: Sedlák, Jirí
(86) International application number: PCT/IB2018/057981
(87) International publication number: WO 2019/077473

(56) References cited:
- WO-A2-2008/074267
- XIA LU ET AL: "An upconversion nanoparticle - Zinc phthalocyanine based nanophotosensitizer for photodynamic the", BIOMATERIALS, ELSEVIER SCIENCE PUBLISHERS BV., BARKING, GB, vol. 35, no. 13, 14 February 2014 (2014-02-14), pages 4146-4156, XP028668205, ISSN: 0142-9612, DOI: 10.1016/J.BIOMATERIALS.2014.01.068
- DEV K CHATTERJEE ET AL: "Upconverting nanoparticles as nanotransducers for photodynamic therapy in cancer cells", NANOMEDICINE, vol. 3, no. 1, 1 February 2008 (2008-02-01), pages 73-82, XP055555609, GB ISSN: 1743-5889, DOI: 10.2217/17435889.3.1.73
- HAI SHENG QIAN ET AL: "Mesoporous-Silica-Coated Up-Conversion Fluorescent Nanoparticles for Photodynamic Therapy", SMALL, vol. 5, no. 20, 16 October 2009 (2009-10-16), pages 2285-2290, XP055555441, DE ISSN: 1613-6810, DOI: 10.1002/smll.200900692
- HANJIE WANG ET AL: "MC540 and Upconverting Nanocrystal Coloaded Polymeric Liposome for Near-Infrared Light-Triggered Photodynamic Therapy and Cell Fluorescent Imaging", ACS APPLIED MATERIALS & INTERFACES, vol. 6, no. 5, 18 February 2014 (2014-02-18), pages 3219-3225, XP055490639, US ISSN: 1944-8244, DOI: 10.1021/am500097f
- BO ENA SIKORA ET AL: "Paper;Transport of NaYF4:Er3+, Yb3+ up-converting nanoparticles into HeLa cells;Transport of NaYF4: Er3 +, Yb3 + up-converting nanoparticles into HeLa cells", NANOTECHNOLOGY, IOP, BRISTOL, GB, vol. 24, no. 23, 13 May 2013 (2013-05-13), page 235702, XP020245365, ISSN: 0957-4484, DOI: 10.1088/0957-4484/24/23/235702
- JEROME BARGE ET AL: "Killing efficacy of a new silicon phthalocyanine in human melanoma cells treated with photodynamic therapy by early activation of mitochondrion-mediated apoptosis", EXPERIMENTAL DERMATOLOGY, vol. 13, no. 1, 1 January 2004 (2004-01-01), pages 33-44, XP055486070, COPENHAGEN; DK ISSN: 0906-6705, DOI: 10.1111/j.0906-6705.2004.00147.x
- MEEROVICH I. G. ET AL: "Aluminium hydroxide tetra-3-phenylthiophthalocyanine as new photosensitizer for photodynamic therapy and fluorescent diagnostics", PROC. SPIE, CURRENT RESEARCH ON LASER USE IN ONCOLOGY: 2000-2004, vol. 5973 59730I-1, 7 December 2006 (2006-12-07), XP040215593, ISSN: 1605-7422
- MEEROVICH I G ET AL: "Near-infrared photosensitizers based on nanostructured forms of phthalocyanine derivatives", RUSSIAN JOURNAL OF GENERAL CHEMISTRY, M A I K NAUKA - INTERPERIODICA, RU, vol. 85, no. 1, 17 February 2015 (2015-02-17), pages 280-288, XP035451391, ISSN: 1070-3632, DOI: 10.1134/S1070363215010430 [retrieved on 2015-02-17]

## Description

### Field of Technology

Injection substance containing light-converting substances in the form of nanoparticles and liposomes containing phtalocyanine for photodynamic therapy in infrared radiation range.

### State of the Art

The photodynamic therapy (PDT) used for the treatment of tissue tumours is based on the process where an active substance is accumulated in a tumour tissue due to the higher demands of the tumour tissue for nourishment. The tumour tissue is in some cases quite well perfused, interwoven with fenestrated capillaries and therefore, all substances get to it easier and quickly through the vascular tree. After the active substance is applied and concentrates in the tumour, PDT is used to irradiate the tumour tissue with light of certain wavelength.

A lot of hydrophobic substances have been developed in the world for the photodynamic therapy of tumour diseases, they have been clinically tested (some of them pre-clinically only) for intervals between the application and irradiation (T_{DL}) in the range from one hour up: benzyl ester of delta aminolevulinic acid, Benzvix (T_{DL} 4 to 6 hours) registered in EU for treatment of gastrointestinal tumours, U.S.patent 6492420, (company Photocure, Oslo, Norway); Temoporfin or Foscan (methyl-tetrahydroxyphenyl chlorin, (T_{DL} 96 hours), WO 0166550, Biolitec Pharma, Scotland, United Kingdom) approved in the EU for palliative tumours in the area of head and neck, tumours of prostate and pancreas; derivate of Benzoporfyrin, thus Verteporfin (BPD-MA, Visudyne, Novartis, UK) which is in clinical tests for cutaneous amelanotic tumours; and silicon phtalocyanine also in clinical tests for the treatment of cutaneous tumours, including so called Bowen's disease, actinic ceratosis, with the currently shortest T_{DL} of 1 hour.

The US patent claim US 5616602 describes pharmaceutical composition for topic application of phtalocyanines which due to their properties enter surface tumours and lesions and then they can be irradiated. The US patent US6527759 describes the equipment for application of light activated active substances. By virtue of this equipment, the active substance can be applied and at the same time tumours positioned deep in the tissue irradiated, where the laser light of wavelength commonly used in PDT would not reach. However, the application of active substances is rather complex even if using this equipment.

Other documents could be considered as a prior art, namely:
BIOMATERIALS, 2014, 35(13), 4146-4156 discloses photodynamic therapy by using a saline intratumoral injection of the upconversion nanoparticles based on NaYF₄ nanocrystals doped with Yb3⁺ and Er³⁺ further coated with poly(allylamine) (PAAM), to which zinc phthalocyanine (ZnPC) photosyntetiser is covalently attached.
NANOMEDICINE, 2008, 3(1), 73-82 discloses an upconversion nanoparticle (UCNP) based on NaYF₄ nanocrystals doped with Yb3⁺ and Er³⁺ further coated with polyethyleneimine polymer (PEi), to which Zn PC is covalently attached. The UCNP particles were administered into rats subcutaneously in a form of suspension in PBS.
SMALL, 2009, 5(10), 2285-2290 discloses an upconversion nanoparticle (UCNP) based on NaYF₄ nanocrystals doped with Yb3⁺ and Er³⁺ further coated with mesopourous-silicate loaded with ZnPC. ACS APPL MATINTER, 2014, 6(5) 3219-3225, XP055490639 discloses a polymeric liposome for near-infrared photodynamic therapy loaded with unconverting nanocrystals of NaYF₄:Yb³⁺_Er³⁺ and merocyanine 540 as photosyntetizer, wherein the shell of said liposome consists of three functionalised amphiphilic polymers: octadecyl-quaternized lysine-modified chitosan (OQLCS), folate acid grafted OQLCS and transactivating transduction (TAT) protein grafted OQLCS (TAT-OQLCS).
NANOTECHNOLOGY, 2013, 24(23), 235702 discloses NaYF₄:Yb³⁺_Er³⁺ nanoparticles incorporated into a liposome Lipofectamine 2000 (known as comprising a mixture of DOSPA and DOPE) as a fluorescent probe for imaging.
EXPER DERMAT, 2004, 13(1), 33-44 discloses a liposome based on egg-yolk-lecithin encapsulating a silicon phthalocyanine for photodynamic therapy.
PROC. SPIE, CURRENT RESEARCH ON LASER USE IN ONCOLOGY: 2000-2004, vol. 5973 597301 and RUSSIAN J GEN CHEM, 2015, 85(1), 280-288 disclose a liposome based on lecithin, cholesterol, cardiolipin/ PEG-2000-DSPE encapsulating aluminium phthalocyanine photosensitizers for near-infrared dynamic therapy. The closest cases to our present invention are the patents CZ 298978 and WO 2008/07 4267 A2 respectively, which were a certain step to the current patent application.

The documents disclose a topical liposomal gel for photodynamic therapy comprising lecithin and hydrophobic phthalocyanines. Said documents describe preparation of liposome gel preparation with phtalocyanine for topical application in photodynamic therapy. The disadvantage of that design was bad permeation of radiation with wavelength 670 nm, necessary for activation of phtalocyanine into the tissue. Another disadvantage of the preparation was its gel formulation which also allowed only surface application. Therefore, the preparation was limited to topical administration and irradiation of tumours immediately under the skin.

### Description of the Invention

The solution proposed has appeared after an accidental combination of phtalocyanine liposomes, routinely being prepared for the production of a topical gel preparation and light-converting (up-converting) nanoparticles, being prepared in a wide range in an adjacent workplace. Pilot testing of the drug form has shown a big potential for the application in photodynamic therapy (PDT) of tumour diseases. Currently, undisclosed interaction of nanoparticles with liposome surface occurs in the system liposome-phtalocyanine-nanoparticle of ytterbium doped with Yb³⁺ and Er³⁺ and thus, the whole system travels together in the vascular tree after intravenous administration. Because of higher affinity of tumour tissue the drug form is accumulated very quickly in a tumour, in order of minutes since the administration. After the tumour is irradiated with light of wavelength in infrared (IR) spectrum (most frequently 980 nm) which passes deep into the tissue, due to the up-converting nanoparticles absorption of light of higher wavelength and irradiation of light with shorter wavelength, necessary for activation of phtalocyanines and elimination of tumour tissue.

The benefit of the solution proposed lies in the possibility to apply light with wavelength in IR spectrum which passes deeper into the tissue than the currently used light with wavelength 670 nm. Thus, the proposed drug form allows expansion of indications of infrared photodynamic therapy.

The technical solution concerns the drug form of the injection preparation with up-converting nanoparticles and phtalocyanine liposomes applicable for infrared photodynamic therapy of tumour diseases. The drug form involves:
- liposomes, best if lecithin ones, with size <1 µm,
- hydrophobic form of phtalocyanine, best if hydroxy-aluminium (or hydrophobic aluminium phtalocyanine, hydrophobic zinc phtalocyanine, hydrophobic silicon or organo-silicon phtalocyanine - hereinbelow marked as PCH), in the form of microcrystals with size <1 µm or modified microcrystals (covered with pharmaceutically acceptable sugar, polymer, lipid or salt in ratio 5-10 wt.%) and phtalocyanine is incorporated in the lipid double layer of liposome,
- light converting nanoparticles (after irradiation with light of wavelength in IR spectrum - approximately 980 nm - they emit light with wavelength necessary for activation of phtalocyanines - approximately 630 to 720 nm), best if covered with non-porous silica and usually composed of NaYF₄ doped with Yb³⁺ and Er³⁺, with optimum size around 40 nm.

As we have found that not all commercially available hydroxy-aluminium phtalocyanines have the same effect in the application according to the proposed solution, it was necessary to better specify the active substance. The content of impurities in phtalocyanines has been determined using HPLC to up to 5%, the content of metal in the active substance has been determined by titration to 3.8 to 4.6%, and no sample may contain more than 0.1% of phtalocyanine without a central metal atom. In spite of the fact that the microfluidization process unifies the crystal structure of the active substance and forms pseudosolution, also the form of the used phtalocyanine shows to be important for function of the whole system. phtalocyanine has been transferred into the α form through reprecipitation from solution of concentrated sulphuric acid. Then the product has been neutralised and dried in air to constant weight at 105°C. Preclinical tests have indicated combination of mixture of α and β modifications as the most suitable where the mixture should not contain more than 20 wt.% of P modification. The crystal form has been investigated using powder x-ray diffraction where the forms have shown various response.

Up-converting nanoparticles have been prepared using the microwave method or using the fluoridation method at 240°C. Preferable way was when the nanoparticles have been covered with tetra ethyl orthosilicate (TEOS). Optimisation of preparation of up-converting nanoparticles required finding suitable conditions for preparation of nanoparticles with required properties - size and photophysical properties. With the optimum size, shape and uniformity of nanoparticles the efficiency of up-conversion is maximised, and the nanoparticles are also able to enter the tumour tissue within the drug form, e.g. through fenestred capillaries. Nanoparticles YF₃:YbEr have been prepared using metal chlorides, NaBF₄ and ion fluid bmimCl. The size of nanocrystals formed depends on reaction time, the optimum has shown to be 260 minutes at 240°C with the resulting size of nanocrystals around 40 nm and with the required photophysical properties which also depend on the reaction time. The nanoparticles prepared can be excited with light with wavelength 980 nm where they emit radiation at 670 nm necessary for activation of phtalocyanines.

The liposome drug form with light-converting nanoparticles is prepared in such a way that lecithin (or another lipid) in pharmacological purity in concentration 1 to 40 mg per milliliter of fluid, best if sterile isotonic solution, is fluidised in a microfluidiser in a suitable chamber to final size of particles less than 1000 nanometres under temperature above 0°C and pressure 1000-2000 Bar. Then under permanent stirring the active substance or the adjusted active substance (hydrophobic hydroxy-aluminium phtalocyanine, hydrophobic aluminium phtalocyanine, hydrophobic zinc phtalocyanine, hydrophobic silicon or organo-silicon phtalocyanine or hydrophobic phtalocyanine without metal) in ratio from 0.1:1 to 3:1 to lecithin (lipid) and prepared up-converting nanoparticles in weight ratio 0.1:1 to 0.25:1 to lecithin are added. The resulting suspension again is fluidised in a microfluidiser in a suitable smaller chamber to the final size of particles less than 500 nanometres under pressure 1000 to 2000 Bar and under temperature above 0°C. Another possible option of preparation of liposom drug form with light-converting nanoparticles is fluidization of lecithin or another lipid (in pharmacological purity in concentration 1 to 40 mg per mililiter of fluid, best if sterile isotonic solution) in a microfluidiser in a suitable chamber. The final size of particles is less than 1000 nanometres. The fluidization process runs under pressure 1000 to 2000 Bar, at least, at temperature above 0°C. Then the active substance (hydrophobic hydroxyaluminium phtalocyanine, hydrophobic aluminium phtalocyanine, hydrophobic zinc phtalocyanine, hydrophobic silicon or organo-silicon phtalocyanine or hydrophobic phtalocyanine without metal) or the adjusted active substance (best if covered with glucose, NaCl, polyethylene glycol or lecithin) is fluidised separately in a microfluidiser in a suitable chamber. The active substance is used in ratio from 0.1:1 to 3:1 to lecithin (lipid) in equal volume of the fluid, best if isotonic solution. The final size of particles after fluidization is less than 1000 nanometres under pressure 1000 to 2000 Bar, at least. Then the both fluidized components are mixed together with light-converting nanoparticles in weight ratio 0.1:1 to 0.25:1 to lecithin and are fluidized together in a microfluidiser in a suitable smaller chamber to the final size of particles equal or less than 500 nanometres under pressure 1000 to 2000 Bar, at least, and under temperature above 0°C.

Last but not least the option of preparation of the liposome drug form with light-converting nanoparticles is the procedure where lecithin or another lipid (in pharmacological purity in concentration 1 to 40 mg per millilitre of fluid, best if sterile isotonic solution) is processed using microfluidization together with the active substance (hydrophobic hydroxyaluminium phtalocyanine, hydrophobic aluminium phtalocyanine, hydrophobic zinc phtalocyanine, hydrophobic silicon or organo-silicon phtalocyanine or hydrophobic phtalocyanine without metal) or the adjusted active substance (best if covered with glucose, NaCl, polyethylene glycol or lecithin) in ratio from 0.1:1 to 3:1 to lecithin (lipid). The resulting suspension is then mixed with light-converting nanoparticles in weight ratio 0.1:1 to 0.25:1 to lecithin and all the components together are then fluidized in a microfluidiser in a suitable chamber to the final size of particles equal or less than 500 nanometres at pressure from 1000 to 2000 Bar, at least, at temperature above 0°C. The final size of particles is less than 500 nanometres under pressure 1000 to 2000 Bar, at least, at temperature above 0°C.

Various combinations of lipid processing, light-converting nanoparticles of ytterbium and an active substance using microfluidization under given conditions provide a drug form with similar properties as examples of the invention execution show.

Microfluidization chambers of types Z and Y are used most frequently within the microfluidization process. Labelling of the chambers indicates their geometric shape, it is a long capillary with certain inner diameter, most frequently 100 to 200 nm which contains a host of diamond blades through which the micro fluidized suspension is driven under pressure. Presence of bends and turns in chambers enables mixing of the microfluidized suspension and prevents rise of unrequired laminar flow of the suspension through the capillary.

The resulting liposome drug form with light-converting nanoparticles is applied in therapy of tumours and metastases intravenously. Natural processes of the body transport the preparation and concentrate it in indicated tumours. The process takes minutes from application to concentration of the preparation in the tumour (tumours). Then the tumour is irradiated with light with wavelength 980 nm and intensity 1 J/cm² or more.

The drug form proposed has shown disintegration effect on tumours and induced remission of tumours in preclinical tests in athymic nu/nu mice with xenotransplanted human tumours.

### Summary of presented drawings

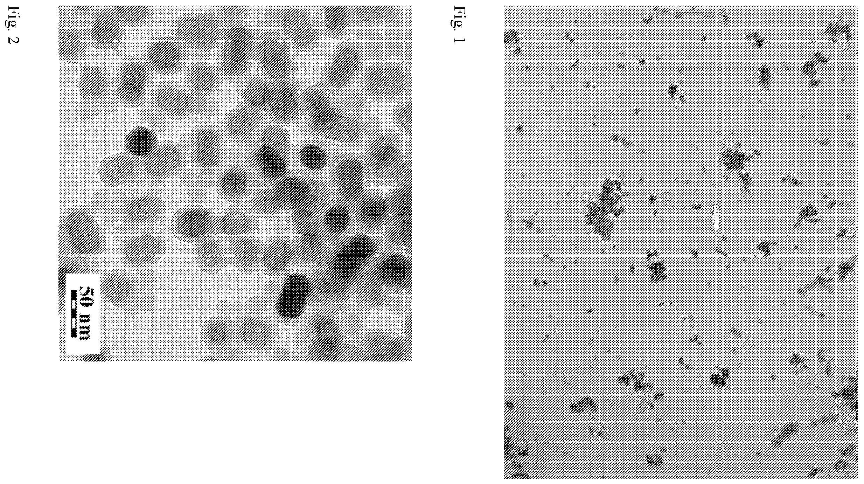

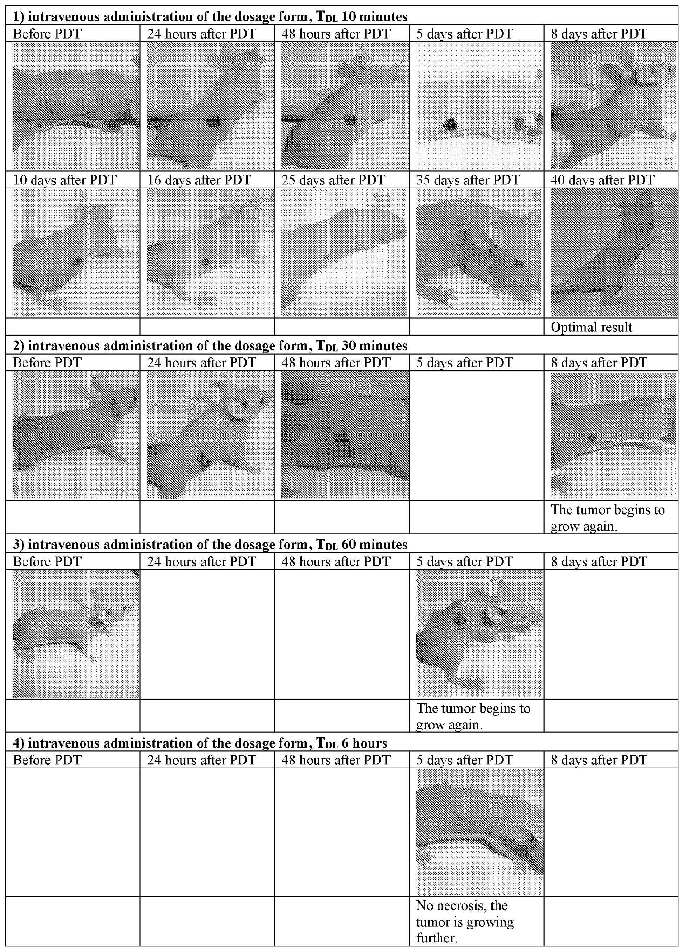

- Fig. 1:: Microscopic check of microfluidization efficiency. Dark small crystals are non-incorporated phtalocyanine, transparent bodies are liposomes.
- Fig. 2:: Up-converting nanoparticle covered with non-porous silica. Photo from electron microscope.
- Fig. 3:: Result of infrared PDT for various T_{DL} (1-5), for a drug form without PCH (6), for application of only nanoparticles (7).
- Fig. 4:: Comparison of change of tumour volume when applying various drug forms with PCH according to examples 7a-9b intratumourously. T_{DL} 10 minutes.
- Fig. 5:: Comparison of change of tumour volume when applying various drug forms with PCH. T_{DL} 10 minutes. A control group of xenotransplanted mice without treatment ("control"), a group of mice treated with gel topical preparation according to patent CZ 298978 ("fc+lipo, topical"), a group of mice treated with the drug form according to this patent intratumourously ("fc+lipo+nano i.t."), a group of mice treated with the drug form according to this patent intravenously (fc+lipo+nano i.v.").
- Fig. 6:: Comparison of size of up-converting nanoparticles prepared according to example 5 as depending on water content in the reaction mixture.
- Fig. 7:: Comparison of size of up-converting nanoparticles prepared according to example 6 as depending on reaction time and ratio of sodium to mixture of chlorides.
- Fig. 8:: Comparison of change of tumour volume when applying various drug forms with PCH. T_{DL} 10 minutes. UCN labels up-converting nanoparticles, FC labels phtalocyanine, m1-8 labels individual test mice, C labels control mice only irradiated with laser.

### Examples of Invention Execution

### Examples of procedure of preparation of powder PCH:

### Example 1

Powder finely ground PCH has been covered with glucose in ratio 5 - 10 wt. % to PCH.
a) 50 mg of glucose was mixed with 10 ml of distilled water. Powder hydroxyaluminium phtalocyanine (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered with glucose in ratio of 5% .
b) 75 mg of glucose was mixed with 10 ml of distilled water. Powder hydroxyaluminium phtalocyanine (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered with glucose in ratio of 7.5% .
c) 100 mg of glucose was mixed with 10 ml of distilled water. Powder hydroxyaluminium phtalocyanine (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered with glucose in ratio of 10% .

### Example 2

Powder finely ground PCH has been covered with polyethylene glycol PEG 600 in ratio 5 - 10 wt. % to PCH.
a) 50 mg of polyethylene glycol PEG 600 was mixed with 10 ml of distilled water. Powder hydroxyaluminium phtalocyanine (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered PEG in ratio of 5%.
b) 75 mg of polyethylene glycol PEG 600 was mixed with 10 ml of distilled water. Powder hydroxyaluminium phtalocyanine (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered PEG in ratio of 7.5%.
c) 100 mg of polyethylene glycol PEG 600 was mixed with 10 ml of distilled water. Powder hydroxyaluminium phtalocyanine (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered PEG in ratio of 10%.

### Example 3

Powder finely ground PCH has been covered with lecithin in pharmacological quality in ratio 5 - 10 wt. % to PCH.
a) 50 mg of powder lecithin was mixed with 10 ml of ethanol heated to 40°C. Powder hydroxyaluminium PCH (1g) with size of particles ranging from 250 to 500 nm was added to the suspension under permanent stirring and the complete mixture was mixed at laboratory temperature for 10 minutes, at least. Then the suspension was heated to temperature 37°C and all the water was evaporated gradually. The result was PCH covered with lecithin in ratio of 5%.
b) 75 mg of powder lecithin was mixed with 10 ml of ethanol heated to 40°C. Powder hydroxyaluminium PCH (1g) with size of particles ranging from 250 to 500 nm was added to the suspension under permanent stirring and the complete mixture was mixed at laboratory temperature for 10 minutes, at least. Then the suspension was heated to temperature 37°C and all the water was evaporated gradually. The result was PCH covered with lecithin in ratio of 7.5%.
c) 100 mg of powder lecithin was mixed with 10 ml of ethanol heated to 40°C. Powder hydroxyaluminium PCH (1g) with size of particles ranging from 250 to 500 nm was added to the suspension under permanent stirring and the complete mixture was mixed at laboratory temperature for 10 minutes, at least. Then the suspension was heated to temperature 37°C and all the water was evaporated gradually. The result was PCH covered with lecithin in ratio of 10%.

### Example 4

a) Powder finely ground PCH has been covered with NaCl in ratio 5 - 10 wt. % to PCH. 50 mg of NaCl was mixed with 10 ml of distilled water. Powder hydroxyaluminium PCH (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered with NaCl in ratio of 5%.
b) 75 mg of NaCl was mixed with 10 ml of distilled water. Powder hydroxyaluminium PCH (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered with NaCl in ratio of 7.5%.
C) 100 mg of NaCl was mixed with 10 ml of distilled water. Powder hydroxyaluminium PCH (1g) with size of particles ranging from 250 to 500 nm was added to the solution under permanent stirring and the complete mixture was mixed at laboratory temperature for 10 minutes, at least. Then the suspension was heated to temperature 37°C and all the water was evaporated gradually. The result was PCH covered with NaCl in ratio of 10%.

### Examples of production of up-converting nanoparticles:

### Example 5

Into a 25ml teflon vessel, 80 mg of mixture of chlorides of lanthanoids (YCl₃, YbCl₃, ErCl₃, GdCl₃ in the form of hexahydrates, in molar ratio of elements Y:Yb:Er:Gd = 80:16:2:2) was placed and it was mixed with 300 mg of NaOH. 500 to 6000 mg of distilled water and 1 ml (1050 mg) of ion fluid l-Butyl-3-methylimidazolium chloride (bmimCl) melted at 50°C was added into the vessel. The mixture was stirred for 5 minutes. Then 30 mg of NH₄F was added and the mixture was stirred for next 2 minutes. Then the vessel was placed into a microwave oven DAEVO KOC-1B5K. The reaction ran at power 1000 W for 1 minute. After the reaction took place, the mixture was moved into 20 ml of methanol and the nanoparticles were separated using centrifugation. The product was refined by washing in 10 ml of methanol and 10 ml of hot distilled water.

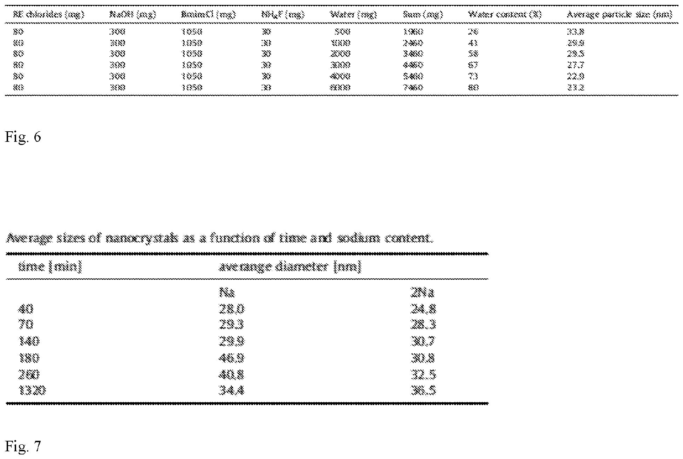

The amount of distilled water added into the reaction mixture has an effect on the size of the crystals. The more water, the lesser outgoing nanoparticles. Higher water content in the reaction mixture results in quicker heating of the complete mixture and thus in quicker fluoridation. Increasing the weight of water in the reaction mixture above 4000 mg has no significant effect on the size of particles (see Figure 6).

The up-converting nanoparticles acquired of NaYF₄ doped with Yb³⁺ (20% doping, in relation to the quantity of Y) and Er³⁺ (2.5% doping, in relation to the quantity of Y) and Gd³⁺ (2.5% doping, in relation to the quantity of Y) have shown the required photochemical properties but they have not shown uniformity of the particles required.

### Example 6

Ion fluid bmimCl (l-Butyl-3-methylimidazolium chloride) was melted first in a vessel of fluoropolymer (PFA) at temperature 60°C. Hexahydrates of chlorides YCl₃, YbCl₃ and ErCl₃ were mixed in molar ratio of elements Y:Yb:Er = 80:18:2. NaBF₄ was used as a fluoridation agent, it was added to the mixture of chlorides in stoichiometric ratio 1:1 or 2:1, sodium to mixture of chlorides. The prepared powder was added to the melted ion fluid, and the complete mixture was stirred. Then the mixture was placed in a drying kiln preheated to 240°C for 40, 70, 140, 180, 260 minutes and 22 hours.

The reaction time and the ratio of sodium to chlorides mixture had an effect on the size of the prepared nanoparticles and also on the quantum yield of up-conversion and thus on photochemical properties of nanoparticles. The optimum reaction time showed to be 260 minutes at temperature 240°C with the resulting nanoparticles size around 40 nm and an efficient up-conversion of light with the wavelength 980 nm to the emission wavelength 670 nm. The optimum up-conversion has also been achieved for the ratio of sodium to mixture of chlorides 1:1. The required properties of nanoparticles were also acquired for reaction time of 22 hours at temperature 240°C and ratio of sodium to the mixture of chlorides 2:1. Because of the time consuming nature of the whole procedure, nanoparticles prepared under the following conditions were chosen for preparation of the drug form: 240°C, 260 minutes, 1:1 sodium: mixture of chlorides. The overview of reaction time influence and the ratio of sodium to the mixture of chlorides on the nanoparticles size is presented in Figure 7.

A part of nanoparticles of NaYF₄ acquired doped with Yb³⁺ (22.5% doping, in relation to quantity of Y) and Er³⁺ (2.5% doping, in relation to quantity of Y) was further adjusted being covered by a silicate polymer layer using tetraethylorthosilicate (TEOS). 6.6 ml of Triton X-100 (Sigma Aldrich), 6.6 ml of HNO₃ (1M), 32.6 ml of acetylacetone, 88 ml of TEOS and 66 ml of ethanol was mixed in a plastic vessel to create a layer-forming solution. This solution was mixed for 6 hours. 0.4 g of prepared nanoparticles was added into 25 ml of the layer-forming solution being mixed. The suspension was stirred in a laboratory hood at laboratory temperature till the layer-forming solution evaporated. The result of covering were up-converting nanoparticles of NaYF₄ doped with Yb³⁺ (22.5% doping, in relation to quantity of Y) and Er³⁺ (2.5% doping, in relation to quantity of Y) covered with non-porous silica.

### Examples of microfluidization procedure:

### Example 7

a) Powder lecithin in pharmacological purity in concentration of 10 mg per millilitre of sterile isotonic solution, 100mM potassium-phosphate buffer (KPI) was first fluidized in a cooled microfluidizer (manufactured by company Microfluidics, Inc., USA, for pilot operations). Fluidization was carried out in a chamber of the Z type with diameter of 100 micrometres in 60 cycles in the way so that the whole volume of the fluid passed several times through the microfluidization chamber at pressure 1000 Bar. Then light-converting nanoparticles prepared in example 5 were added under permanent stirring and while using 500 mg of water, in weight ratio 0.2:1 to lecithin, and then non-adjusted microcrystalline powder phtalocyanine (PCH with size particles ranging from 250 to 500 nm) in weight ratio 1:1 to lecithin was added under permanent stirring and heating the whole mixture to 35°C. Then the suspension was processed again in a microfluidization chamber of the Z type with diameter of 100 micrometres, namely in 100 cycles of passing fluid through the chamber at pressure of 1500 Bar and under continuous cooling with crumbled ice.
   34.7 mg/ml of the drug form 7a with concentration of hydroxyaluminium PCH was prepared.
b) Powder lecithin in pharmacological purity in concentration of 30 mg per millilitre of sterile isotonic solution KPI was first fluidized in a cooled microfluidizer (manufactured by company Microfluidics, Inc., USA, for pilot operations). Fluidization was carried out in a chamber of the Y type with diameter of 100 micrometres in 60 cycles in the way so that the whole volume of the fluid passed several times through the microfluidization chamber, at pressure of 1000 Bar. Then light-converting nanoparticles prepared in example 6, covered with TEOS, in weight ratio 0.15:1 to lecithin, were added under permanent stirring and then microcrystalline powder PCH covered with glucose prepared in example 1b was added under permanent stirring and heating the whole mixture to 37°C, and the active substance had weight ratio 2:1 to lecithin. Then the suspension was processed again in a microfluidization chamber of the Y type with diameter 100 of micrometres, namely in 110 cycles of passing fluid through the chamber at pressure of 1600 Bar and under continuous cooling with crumbled ice.
   36.2 mg/ml of the drug form 7b with concentration of hydroxyaluminium PCH was prepared.

The efficiency of the whole process was followed in a microscope so that dark blue crystals of phtalocyanine would not appear in the resulting mixture any more, and all phtalocyanine would be incorporated into liposomes or would be washed off from the drug form.

### Example 8

a) Powder lecithin in pharmacological purity in concentration of 10 mg per millilitre of sterile isotonic physiological solution was first fluidized in a cooled microfluidizer (manufactured by company Microfluidics, Inc., USA, for pilot operations). Fluidization was carried out in a chamber of the Z type with diameter of 100 micrometres in 60 cycles in the way so that the whole volume of the fluid passed several times through the microfluidization chamber at pressure 1200 Bar. Then non-adjusted microcrystalline powder PCH (with size particles ranging from 250 to 500 nm, in weight ratio 2:1 to lecithin in the same volume of isotonic solution) was fluidized independently, namely in a microfluidization chamber of the Y type with diameter of 100 micrometres in 50 cycles and then in one with diameter of 75 micrometres also in 50 cycles, at least, together in 100 cycles of passing fluid through the chamber at pressure of 1500 Bar. Both the fluidized components were preheated to temperature 37°C, mixed and light-converting nanoparticles prepared in example 6, not-covered, in weight ratio 0.1:1 to lecithin were added under permanent stirring. Then the whole mixture was again processed in a microfluidization chamber of the Z type with diameter of 100 micrometres, in total 100 cycles of passing fluid through the chamber at pressure of 2000 Bar under continuous cooling with crumbled ice.
   33.6 mg/ml of the drug form 8a with concentration of hydroxyaluminium PCH was prepared.
b) Powder lecithin in pharmacological purity in concentration of 30 mg per millilitre of sterile isotonic solution was first fluidized in a cooled microfluidizer (manufactured by company Microfluidics, Inc., USA, for pilot operations). Fluidization was carried out in a chamber of the Y type with diameter of 100 micrometres in 50 cycles in the way so that the whole volume of the fluid passed several times through the microfluidization chamber, at pressure of 1500 Bar. Then microcrystalline powder PCH covered with NaCl prepared in example 4a, in weight ratio 1:1, active substance in relation to lecithin in the same volume of isotonic solution) was fluidized independently, namely in a microfluidization chamber of the Z type with diameter of 75 micrometres, in total 100 cycles of passing fluid through the chamber at pressure of 1600 Bar. Both the fluidized components were mixed after heating to 37°C and under permanent stirring light-converting nanoparticles prepared in example 5 were added while using 4000 mg of water, in weight ratio 0.15:1 to lecithin. Then the whole mixture was again processed in a microfluidization chamber of the Z type with diameter of 100 micrometres, namely in total 120 cycles of passing fluid through the chamber at pressure of 2000 Bar under continuous cooling with crumbled ice.
   33.1 mg/ml of the drug form 8b with concentration of hydroxyaluminium PCH was prepared.

The efficiency of the whole process was followed in a microscope so that dark blue crystals of phtalocyanine would not appear in the resulting mixture any more, and all phtalocyanine would be incorporated into liposomes or would be washed off from the drug form.

### Example 9

a) Powder lecithin in pharmacological purity in concentration of 10 mg per millilitre of sterile isotonic solution KPI was first fluidized in a cooled microfluidizer (manufactured by company Microfluidics, Inc., USA, for pilot operations). Fluidization was carried out in a chamber of the Z type with diameter of 100 micrometres in 60 cycles in the way so that the whole volume of the fluid passed several times through the microfluidization chamber, at pressure of 1000 Bar. Then light-converting nanoparticles prepared according to example 6, not-covered, in weight ratio 0.25:1 to lecithin, were added to fluidized lecithin under permanent stirring. Non-adjusted microcrystalline powder PCH in weight ratio 2:1 to lecithin was added to the mixture under permanent stirring. Then the suspension was processed in a microfluidization chamber of the Y type with diameter of 100 micrometres in 60 cycles and then in one with diameter of 75 micrometres in 40 cycles, at least, together 100 cycles, at least, of passing fluid through the chamber at pressure of 1700 Bar and under continuous cooling with crumbled ice. Then the substance was processed in a microfluidization chamber of the Z type with diameter of 100 micrometres, namely in total 100 cycles of passing fluid through the chamber at pressure of 2000 Bar and under cooling with crumbled ice.
   35.9 mg/ml of the drug form 9a with concentration of hydroxyaluminium PCH was prepared.
b) First powder lecithin in pharmacological purity in concentration of 30 mg per millilitre of sterile isotonic solution KPI was fluidized in a cooled microfluidizer (manufactured by company Microfluidics, Inc., USA, for pilot operations). Fluidization was carried out in a chamber of the Z type with diameter of 100 micrometres in 50 cycles in the way so that the whole volume of the fluid passed several times through the microfluidization chamber, at pressure of 1200 Bar. Then light-converting nanoparticles prepared in example 6, covered with TEOS, in weight ratio 0.1:1 to lecithin g were added to fluidized lecithin under permanent stirring. Microcrystalline powder PCH covered with polyethylene glycol prepared in example 2c) in weight ratio 0,5:1, active substance in relation to lecithin, was added to the mixture under permanent stirring and heating to temperature 30°C. Then the suspension was processed in a microfluidization chamber of the Y type with diameter of 100 micrometres, namely in total in 100 cycles of passing fluid through the chamber at pressure of 1800 Bar and under continuous cooling with crumbled ice. Then the substance was processed in a microfluidization chamber of the Z type with diameter of 75 micrometres in total 100 cycles of passing fluid through the chamber at pressure of 2000 Bar and under cooling with crumbled ice.
   33.8 mg/ml of the drug form 9b with concentration of hydroxy aluminium PCH was prepared.

The efficiency of the whole process was followed in a microscope so that dark blue crystals of phtalocyanine would not appear in the resulting mixture any more, and all phtalocyanine would be incorporated into liposomes or would be washed off from the drug form.

### Example 10

a) First powder lecithin in pharmacological purity in concentration of 10 mg per millilitre of sterile saline solution was fluidized in a cooled microfluidizer (manufactured by company Microfluidics, Inc., USA, for pilot operations). Fluidization was carried out in a chamber of the Z type with diameter of 100 micrometres in 60 cycles in the way so that the whole volume of the fluid passed several times through the microfluidization chamber, at pressure of 1200 Bar. Then non-adjusted microcrystalline powder PCH in weight ratio 2:1 to lecithin was fluidized independently. Fluidization was carried out in a chamber of the Y type with diameter of 100 micrometres in 40 cycles in the way so that the whole volume of the fluid passed several times through the microfluidization chamber, at pressure of 1000 Bar. Fluidized lecithin was mixed with fluidized PCH, and to their mixture light-converting nanoparticles prepared according to example 6, covered with TEOS, in weight ratio 0.25:1 to lecithin, were added under permanent stirring. Then the suspension was processed in a microfluidization chamber of the Y type with diameter 100 micrometres in total 100 cycles of passing fluid through the chamber at pressure of 2000 Bar and under cooling with crumbled ice.
   35.1 mg/ml of the drug form 10a with concentration of hydroxyaluminium PCH was prepared.
b) First powder lecithin in pharmacological purity in concentration of 30 mg per millilitre of sterile saline solution was fluidized in a cooled microfluidizer (manufactured by company Microfluidics, Inc., USA, for pilot operations). Fluidization was carried out in a chamber of the Z type with diameter of 100 micrometres in 50 cycles in the way so that the whole volume of the fluid passed several times through the microfluidization chamber, at pressure of 1500 Bar. Then microcrystalline powder PCH covered with polyethylene glycol prepared in example 2a in weight ratio 2:1 to lecithin was fluidized independently. Fluidization was carried out in a chamber of the Z type with diameter of 100 micrometres in 60 cycles in the way so that the whole volume of the fluid passed several times through the microfluidization chamber, at pressure of 1200 Bar. Fluidized lecithin was mixed with fluidized PCH and to their mixture light-converting nanoparticles prepared according to example 6, covered with TEOS, in weight ratio 0.2:1 to lecithin, were added under permanent stirring. Then the suspension was processed in a microfluidization chamber of the Z type with diameter of 100 micrometres, namely in 60 cycles of passing fluid through the chamber at pressure of 1500 Bar and then in a chamber of the Y type with diameter of 75 micrometres in 40 cycles, and under cooling with crumbled ice.
   34.2 mg/ml of the drug form 10b with concentration of hydroxy aluminium PCH was prepared.

The efficiency of the whole process was followed in a microscope so that dark blue crystals of phtalocyanine would not appear in the resulting mixture any more, and all phtalocyanine would be incorporated into liposomes or would be washed off from the drug form.

### Example 11

a) First powder lecithin in pharmacological purity in concentration of 10 mg per millilitre of sterile saline solution together with powder microcrystalline PCH covered with NaCl prepared in example 4b, where the active substance had the weight ratio 1:1 to lecithin, was fluidized in a cooled microfluidizer (manufactured by company Microfluidics, Inc., USA, for pilot operations). Fluidization was carried out in a chamber of the Y type with diameter of 100 micrometres in 60 cycles in the way so that the whole volume of the fluid passed several times through the microfluidization chamber, at pressure of 1200 Bar. Then light-converting nanoparticles prepared in example 6, covered with TEOS, in weight ratio 0.1:1 to lecithin, were added under permanent stirring. Then the suspension was processed in a microfluidization chamber of the Z type with diameter 100 micrometres in total 100 cycles of passing fluid through the chamber at pressure of 1800 Bar and under continuous cooling with crumbled ice.
   34.2 mg/ml of the drug form 11a with concentration of hydroxyaluminium PCH was prepared.
b) First powder lecithin in pharmacological purity in concentration of 30 mg per millilitre of sterile isotonic solution KPI together with powder microcrystalline PCH covered with glucose prepared in example 1b, where the active substance had the weight ratio 2:1 to lecithin, was fluidized in a cooled microfluidizer (manufactured by company Microfluidics, Inc., USA, for pilot operations). Fluidization was carried out in a chamber of the Y type with diameter 100 micrometres in 60 cycles in the way so that the whole volume of the fluid passed several times through the microfluidization chamber, at pressure of 1000 Bar. Then light-converting nanoparticles prepared in example 6, covered with TEOS, in weight ratio 0.15:1 to lecithin, were added under permanent stirring. Then the suspension was again processed in a microfluidization chamber of the Y type with diameter of 100 micrometres, in total 110 cycles of passing fluid through the chamber at pressure of 1500 Bar and under continuous cooling with crumbled ice.
   35.2 mg/ml of the drug form 11b with concentration of hydroxyaluminium PCH was prepared.

The efficiency of the whole process was followed in a microscope so that dark blue crystals of phtalocyanine would not appear in the resulting mixture any more, and all phtalocyanine would be incorporated into liposomes or would be washed off from the drug form.

### Examples of testing therapeutical efficiency of prepared drug forms:

### Example 12

Intratumoural application of the drug form directly into a tumour of athymic naked nu/nu mice with xenotransplanted human tumours, actually with amelanotic non-pigmented melanoma of C-32 line, was selected for the purposes of pilot testing (screening) of drug forms prepared in examples 7-11. After implantation of human tumour cells into subcutis of athymic nu/nu mice, the tumour was monitored and as soon as it reached the size of approximately 100-150 mm³ (some 8 days after the transplantation), the intratumoural application of the drug form was executed. The drug form prepared according to examples 7-11 was first filtered through filters with 1 micrometre pores, then 200 microliters of the drug form was applied by injection directly into the tumour of the test mice. After 10 minutes time (T_{DL}) the tumour tissue was irradiated with laser at wavelength 980 nm at output of 3W and total energy 160 J/cm².

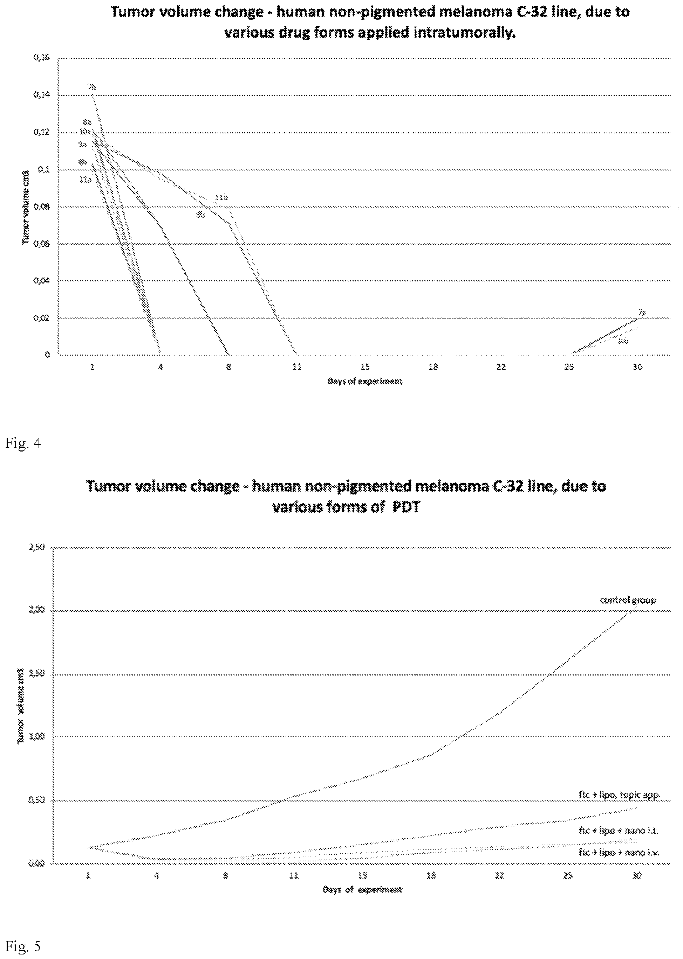

The outcome of the experiment was assessed according to the change of tumour volume and the size of surface necrosis. All the drug forms prepared according to examples 7-11 showed similar results in intratumoural application (see Figure 4). However, only one drug form could be selected for subsequent testing of therapeutical efficiency, namely due to the high number of test animals, and so the drug form prepared according to example 7b was selected at random.

### Example 13

Athymic naked nu/nu mice with xenotransplanted human tumours, actually with amelanotic non-pigmented melanoma of line C-32, were selected for testing of therapeutical efficiency of the liposome drug form. After implantation of human tumour cells into subcutis of athymic nu/nu mice, the tumour was monitored and as soon as it reached the size of approximately 200 mm³ (some 10 days after the transplantation), the intravenous application of the drug form was executed. The drug form prepared according to example 7b was first filtered through filters with 1 micrometre pores, then 200 microlitres of the drug form was applied by injection into a tail vein of the test mice. After various intervals of time the tumour tissue was irradiated with laser at wavelength 980 nm at output of 3W and total energy 160 J/cm².

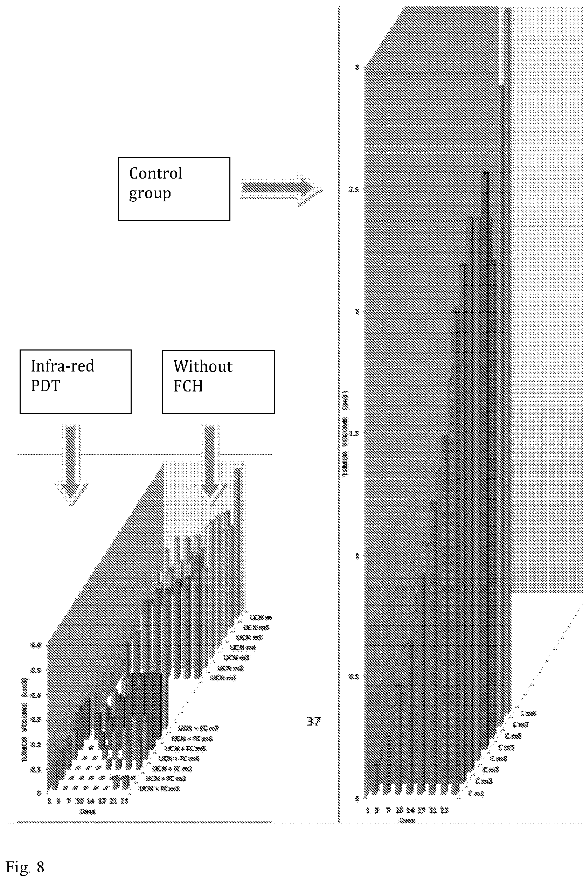

The mice showed remission of tumours and apparent healing accompanied with surface necroses after the treatment. Just these two parameters were used for assessment of the experiment: the size of the tumour in the time after irradiation and the size of surface necroses. Athymic naked nu/nu mice with xenotransplanted human tumours irradiated with laser only, mice with the application of the drug form without irradiation or with the application of the drug form without PCH and subsequent irradiation were used as the control panel. The test results are presented in Figure 8.

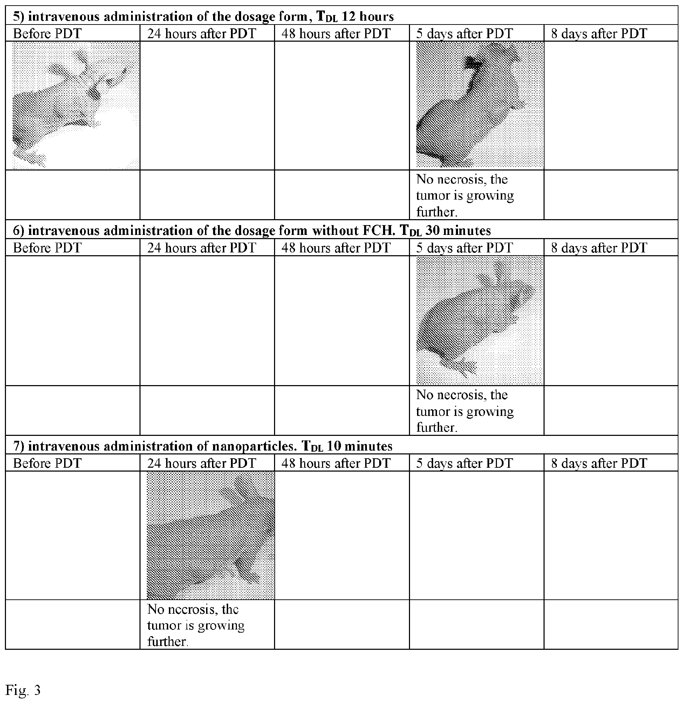

It was established that efficiency of PDT, the size of necroses and the size of a tumour depends on T_{DL}-interval between application of the preparation and irradiation by laser, and the best results were achieved for application of the drug form 10 minutes before laser irradiation, on the other hand, the treatment was ineffective, the tumour was not affected and it grew on in a case of application of the drug form 12 hours before the laser irradiation (see Figure 3). The graph of therapeutic efficiency of the drug form administered i.v. in comparison with the control group, with gel topical preparation according to patent CZ 298978 and with application of the drug form i.t. according to example 10 is presented in Figure 5.

### Applicability in Industry

Liposome drug form with light-converting nanoparticles is applicable in infrared photodynamic therapy of tumour diseases.

## Claims

1. A liposome drug form with light-converting nanoparticles **characterized by** the fact that it contains lecithin, light-converting nanoparticles of NaYF₄ doped with Yb³⁺ and Er³⁺ in weight ratio 0.1:1 to 0.25:1 to lecithin and an active substance in weight ratio 0.1:1 to 3:1 to lecithin, and the active substance is selected in the group involving hydrophobic hydroxyaluminium phtalocyanine, hydrophobic aluminium phtalocyanine, hydrophobic zinc phtalocyanine, hydrophobic silicon or organo-silicon phtalocyanine.

2. The liposome drug form according to claim 1 **characterized by** the fact that light-converting nanoparticles of NaYF₄ doped with Yb³⁺ and Er³⁺ have molar ratio of sodium to ytterbium and erbium 1:1 to 2:1.

3. The liposome drug form according to claim 1 **characterized by** the fact that light-converting nanoparticles of NaYF₄ doped with Yb³⁺ and Er³⁺ have molar ratio of yttrium to ytterbium to erbium 80:18:2.

4. The liposome drug form according to claim 1 or 2 or 3 **characterized by** the fact that light-converting nanoparticles of NaYF₄ doped with Yb³⁺ and Er³⁺ are covered with non-porous silica.

5. The liposome drug form according to claim 1 **characterized by** the fact that the active substance is in the form of microcrystals or modified microcrystals that are covered with pharmaceutically acceptable sugar, polymer, lipid or salt that forms 5 to 10 wt. % of the modified microcrystal.

6. A method of preparation of the liposome drug form according to claim 1 **characterized by** the fact that lecithin and the active substance are mixed with water or aqueous solution in concentration of 10 to 30 mg per millilitre and the suspension formed is applied in a microfluidizer fitted with a microfluidization chamber with diameter of 75 to 100 micrometres under temperature above 0°C, the microfluidizer is set to pressure 100 to 200 MPa and the fluidization process runs for 40 cycles, at least, of transfer of the suspension through the microfluidization chamber, then light-converting nanoparticles of NaYF₄ doped with Yb³⁺ and Er³⁺ are added to the suspension.

7. The method of preparation of the liposome drug form according to claim 6 **characterized by** the fact that the suspension with light-converting nanoparticles of NaYF₄ is fluidized in 50 cycles, at least.

8. The method of preparation of the liposome drug form according to claim 6 **characterized by** the fact that lecithin is mixed with water or aqueous solution in concentration of 10 to 30 mg per millilitre before being mixed with the active substance and the suspension formed is applied in a microfluidizer fitted with a microfluidization chamber with diameter of 100 micrometres under temperature above 0°C, the microfluidizer is set to pressure 100 to 200 MPa and the fluidization process runs for 50 cycles, at least, of transfer of the suspension through the microfluidization chamber.

9. The method of preparation of the liposome drug form according to claim 6 **characterized by** the fact that the active substance is mixed with water or aqueous solution in concentration of 10 to 30 mg per millilitre before being mixed with the lecithin and the mixture formed is applied in a microfluidizer fitted with a microfluidization chamber with diameter of 75 to 100 micrometres under temperature above 0°C, the microfluidizer is set to pressure 100 to 200 MPa and the fluidization process runs for 40 cycles, at least, of transfer of the suspension through the microfluidization chamber.

10. The method of preparation of the liposome drug form according to claims 7 or 8 or 9 or 10 **characterized by** the fact that the aqueous solution is sterile isotonic solution, saline solution or buffer.

11. The liposome drug form according to claim 1 for use as a drug.

12. The liposome drug form according to claim 1 for use in treatment of tumour diseases using infrared photodynamic therapy.

## Patentansprüche

1. Eine liposomale Arzneiform mit lichtkonvertierenden Nanopartikeln, **dadurch gekennzeichnet, dass** sie Lecithin, lichtkonvertierende Nanopartikel von NaYF₄, dotiert mit Yb³⁺ und Er³⁺ im Gewichtsverhältnis 0,1:1 bis 0,25:1 zu Lecithin und einen Wirkstoff im Gewichtsverhältnis 0,1:1 bis 3:1 zu Lecithin enthält, und der Wirkstoff ist ausgewählt aus der Gruppe bestehend aus hydrophobem Hydroxyaluminiumphthalocyanin, hydrophobem Aluminiumphthalocyanin, hydrophobem Zinkphthalocyanin, hydrophobem Silizium- oder siliziumorganischem Phtalocyanin.

2. Liposomale Arzneiform nach Anspruch 1, **dadurch gekennzeichnet, dass** lichtkonvertierende Nanopartikel von NaYF₄, dotiert mit Yb³⁺ und Er³⁺, ein Molverhältnis von Natrium zu Ytterbium und Erbium von 1:1 bis 2:1 aufweisen.

3. Liposomale Arzneiform nach Anspruch 1, **dadurch gekennzeichnet, dass** lichtkonvertierende Nanopartikel von NaYF₄, dotiert mit Yb³⁺ und Er³⁺ ein Molverhältnis von Yttrium zu Ytterbium zu Erbium von 80:18:2 aufweisen.

4. Liposomale Arzneiform nach Anspruch 1 oder 2 oder 3, **dadurch gekennzeichnet, dass** lichtkonvertierende Nanopartikel von NaYF₄, dotiert mit Yb3⁺ und Er³⁺ mit nichtporösem Siliziumdioxid bedeckt sind.

5. Liposomale Arzneiform nach Anspruch 1, **dadurch gekennzeichnet, dass** der Wirkstoff in Form von Mikrokristallen oder modifizierten Mikrokristallen vorliegt, die mit pharmazeutisch akzeptablem Zucker, Polymer, Lipid oder Salz bedeckt sind, das 5 bis 10 Gew.-*%* des modifizierten Mikrokristalls bildet.

6. Verfahren zur Herstellung der liposomalen Arzneiform nach Anspruch 1, **dadurch gekennzeichnet, dass** Lecithin und der Wirkstoff mit Wasser oder wässriger Lösung in einer Konzentration von 10 bis 30 mg pro Milliliter vermischt werden und die gebildete Suspension in einem, mit einer Mikrofluidisierungskammer mit einem Durchmesser von 75 bis 100 Mikrometer ausgestatteten Mikrofluidisierer bei einer Temperatur über 0 °C appliziert wird, der Mikrofluidisator auf einen Druck von 100 bis 200 MPa eingestellt ist und der Fluidisierungsprozess mindestens 40 Zyklen des Transfers der Suspension durch die Mikrofluidisierungskammer dauert, und dann der Suspension lichtkonvertierende Nanopartikel von NaYF₄, dotiert mit Yb³⁺ und Er³⁺, zugesetzt werden.

7. Verfahren zur Herstellung der liposomalen Arzneiform nach Anspruch 6, **dadurch gekennzeichnet, dass** die Suspension mit lichtkonvertierenden Nanopartikeln von NaYF₄ in mindestens 50 Zyklen fluidisiert wird.

8. Verfahren zur Herstellung der liposomalen Arzneiform nach Anspruch 6, **dadurch gekennzeichnet, dass** Lecithin mit Wasser oder wässriger Lösung in einer Konzentration von 10 bis 30 mg pro Milliliter gemischt wird, bevor es mit dem Wirkstoff vermischt wird, und die gebildete Suspension in einem, mit einer Mikrofluidisierungskammer mit einem Durchmesser von 100 Mikrometern ausgestatteten Mikrofluidisator bei einer Temperatur über 0 °C appliziert wird, der Mikrofluidisator auf einen Druck von 100 bis 200 MPa eingestellt ist und der Fluidisierungsprozess mindestens 50 Zyklen des Transfers der Suspension durch die Mikrofluidisierungskammer dauert.

9. Verfahren zur Herstellung der liposomalen Arzneiform nach Anspruch 6, **dadurch gekennzeichnet, dass** der Wirkstoff mit Wasser oder wässriger Lösung in einer Konzentration von 10 bis 30 mg pro Milliliter vermischt wird, bevor er mit dem Lecithin vermischt wird, und das gebildete Gemisch in die einem, mit einer Mikrofluidisierungskammer mit einem Durchmesser von 75 bis 100 Mikrometern ausgestattetem Mikrofluidizer bei einer Temperatur über 0 °C appliziert wird, der Mikrofluidizer auf einen Druck von 100 bis 200 MPa eingestellt ist und der Fluidisierungsprozess mindestens 40 Zyklen des Transfers der Suspension durch die Mikrofluidisierungskammer dauert.

10. Verfahren zur Herstellung der liposomalen Arzneiform nach Anspruch 7 oder 8 oder 9 oder 10, **dadurch gekennzeichnet, dass** die wässrige Lösung eine sterile isotonische Lösung, Kochsalzlösung oder Pufferlösung ist.

11. Liposomale Arzneiform nach Anspruch 1 zur Anwendung als Arzneimittel.

12. Liposomale Arzneiform nach Anspruch 1 zur Anwendung bei der Behandlung von Tumorerkrankungen unter Verwendung von Infrarotlicht bei der photodynamischen Therapie.

## Revendications

1. Une forme de médicament liposomé avec des nanoparticules convertissant la lumière **caractérisée en ce qu'**elle contient de la lécithine, des nanoparticules de NaYF₄ pour convertir la lumière, dopées avec Yb³⁺ et Er³⁺ avec un rapport de poids de 0,1:1 à 0,25:1 à la lécithine et une substance active avec un rapport de poids de 0,1:1 à 3:1 à la lécithine et la substance active est choisie dans le groupes comprenant de l'hydroxyde de phthalocyanine d'aluminium hydrophobe, du phthalocyanine d'aluminium hydrophobe, du phthalocyanine de zinc hydrophobe, du silicium hydrophobe ou du phtalocyanine de silicium organique.

2. La forme de médicament liposomé selon la revendication 1, **caractérisée par le fait que** les nanoparticules de NaYF₄ convertissant la lumière dopées avec Yb³⁺ et Er³⁺ ont un rapport molaire de sodium à ytterbium et erbium de 1:1 à 2:1.

3. La forme de médicament liposomé selon la revendication 1, **caractérisée par le fait que** les nanoparticules de NaYF₄ convertissant la lumière dopées avec Yb³⁺ et Er³⁺ ont un rapport molaire de yttrium à ytterbium à erbium de 80:18:2.

4. La forme de médicament liposomé selon la revendication 1 ou 2 ou 3, **caractérisée par le fait que** les nanoparticules de NaYF₄ convertissant la lumière dopées avec Yb³⁺ et Er³⁺ sont recouvertes de silice non poreuse.

5. La forme de médicament liposomé selon la revendication 1, **caractérisée par le fait que** la substance active est sous forme de microcristaux ou de microcristaux modifiés qui sont recouverts de sucre, polymères, lipides ou sel acceptables sur le plan pharmaceutique qui font 5 à 10 % du poids du microcristal modifié.

6. Une méthode de préparation du médicament liposomé selon la revendication 1, **caractérisée par le fait que** la lécithine et la substance active sont mélangées avec de l'eau ou une solution aqueuse avec une concentration de 10 à 30 mg par millilitre et la suspension formée est appliquée dans un microfluidiseur équipé d'une chambre de microfluidisation d'un diamètre de 75 à 100 micromètres à une température au-dessus de 0 °C, le microfluidiseur est réglé à une pression de 100 à 200 Mpa et le processus de fluidisation fonctionne pendant 40 cycles, au moins, du transfert de la suspension au travers de la chambre de microfluidisation, puis des nanoparticules de NaYF₄ convertissant la lumière dopées avec Yb³⁺ et Er³⁺ sont ajoutées à la suspension.

7. La méthode de préparation de la forme de médicament liposomé selon la revendication 6, **caractérisée par le fait que** la suspension avec les nanoparticules de NaYF₄ convertissant la lumière est fluidisée en 50 cycles, au moins.

8. La méthode de préparation de la forme de médicament liposomé selon la revendication 6, **caractérisée par le fait que** de la lécithine est mélangée avec de l'eau ou une solution aqueuse avec une concentration de 10 à 30 mg par millilitre avant d'être mélangée avec la substance active et la suspension formée est appliquée dans un microfluidiseur équipé d'une chambre de microfluidisation d'un diamètre de 100 micromètres à une température au-dessus de 0 °C, le microfluidiseur est réglé à une pression de 100 à 200 Mpa et le processus de fluidisation fonctionne pendant 50 cycles, au moins, du transfert de la suspension au travers de la chambre de microfluidisation.

9. La méthode de préparation de la forme de médicament liposomé selon la revendication 6, **caractérisée par le fait que** la substance active est mélangée avec de l'eau ou une solution aqueuse avec une concentration de 10 à 30 mg par millilitre avant d'être mélangée à la lécithine et le mélange est appliqué dans un microfluidiseur équipé d'une chambre de microfluidisation de 75 à 100 micromètres à une température au-dessus de 0 °C, le microfluidiseur est réglé à une pression de 100 à 200 Mpa et le processus de fluidisation fonctionne pendant 40 cycles, au moins, du transfert de la suspension au travers de la chambre de microfluidisation.

10. La méthode de préparation de la forme de médicament liposomé selon les revendications 7 ou 8 ou 9 ou 10, **caractérisée par le fait que** la solution aqueuse est une solution isotonique stérile, une solutions saline ou un tampon.

11. La forme de médicament liposomé selon la revendication 1 pour son application comme médicament.

12. La forme de médicament liposomé selon la revendication 1 pour son application dans le traitement des maladies tumorales au moyen d'une thérapie photodynamique aux infrarouges.
